# EUROPEAN PATENT APPLICATION

(11) **EP 2 353 502 A2**
(43) Date of publication of application: **10.08.2011**
(21) Application number: 11153667.8
(22) Date of filing: 08.02.2011
(51) Int. Cl.: A61B 5/024

(54) **Biological information detector and biological information measuring device**

(30) Priority: 10.02.2010 JP 2010027832
(71) Applicant: Seiko Epson Corporation, Shinjuku-ku Tokyo (JP)
(72) Inventor: Sato, Shigemi, Nagano 392-8502 (JP); Iijima, Yoshitaka, Nagano 392-8502 (JP); Yamashita, Hideto, Nagano 392-8502 (JP)
(74) Representative: HOFFMANN EITLE

(57) **Abstract**

To provide a biological information detector or the like in which the detection accuracy or the measurement accuracy can be increased. A biological information detector includes a light-emitting part (14) for emitting light (R1) directed at a detection site (O) of a test subject; a light-receiving part (16) for receiving light (R1') having biological information, the light (R1') being light (R1) emitted by the light-emitting part (14) and reflected at the detection site (O); and a contact part (19) formed from a material that is transparent with respect to a wavelength of light (R1) emitted by the light-emitting part (14), the contact part (19) having a contact surface (19A) that makes contact with the test subject and an opposing surface (19B) disposed opposite the contact surface (19A). The light-emitting part (14) is installed on the opposing surface (19B).

## Description

### BACKGROUND

### Technical Field

The present invention relates to a biological information detector and a biological information measuring device.

### Background Technology

A biological information measuring device measures human biological. information such as, for example, pulse rate, blood oxygen saturation level, body temperature, or heart rate, and an example of a biological information measuring device is a pulse rate monitor for measuring the pulse rate. Also, a biological information measuring device such as a pulse rate monitor may be installed in a clock, a mobile phone, a pager, a PC, or another electrical device, or may be combined with the electrical device. The biological information measuring device has a biological information detector for detecting biological information, and the biological information detector includes a light-emitting part for emitting light towards a detection site of a test subject (e.g., a user), and a light-receiving part for receiving light having biological information from the detection site.

In Patent Citation 1, there is disclosed a pulse rate monitor (or in a broader sense, a biological information measuring device). A light-receiving part (e.g., a light-receiving part 12 in FIG. 16 of Patent Citation 1) of the pulse rate monitor receives light reflected at a detection site (e.g., dotted line in FIG. 16 of Patent Citation 1) via a diffusion reflection plane (e.g., reflecting part 131 in FIG. 16 of Patent Citation 1). In an optical probe 1 in Patent Citation 1 (or in a broader sense, a biological information detector), a light-emitting part 11 and the light-receiving part 12 overlap in plan view, and the size of the optical probe 1 is reduced.

JP-A 2004-337605 (Patent Citation 1) is an example of the related art.

### SUMMARY

### Problems to Be Solved by the Invention

The light-emitting part 11 and the light-receiving part 12 in Patent Citation 1 are arranged, together with a substrate 15, in an interior of the reflecting part 131. The interior of the reflecting part 131 is filled with a transparent material 142. According to a configuration of such description, a predetermined distance is present between the light-emitting part 11 and the detection site, and the detection accuracy of the biological information detector is poor.

According to several modes of the invention, it is possible to provide a biological information detector and a biological information measuring device in which the detection accuracy or the measurement accuracy can be improved.

### Means Used to Solve the Above-Mentioned Problems

A first aspect of the invention relates to a biological information detector, characterized in including:
a light-emitting part for emitting light directed at a detection site of a test subject;
a light-receiving part for receiving light having biological information, the light emitted by the light-emitting part and reflected at the detection site; and
a contact part formed from a material that is transparent with respect to a wavelength of the light emitted by the light-emitting part, the contact part having a contact surface that makes contact with the test subject and an opposing surface disposed opposite the contact surface; wherein
the light-emitting part is installed on the opposing surface.

According to the first aspect of the invention, the light-emitting part is mounted on the opposing surface of the contact part using, e.g., a bump or another connecting member. Specifically, the distance between the light-emitting part and the detection site of the test subject (e.g., a user) can be reduced. Therefore, the amount of light reaching the detection site increases, and the detection accuracy (i.e., the signal-to-noise ratio) of the biological information detector increases.

According to a second aspect of the invention, a first wiring for the light-emitting part may be arranged on the opposing surface, and the light-emitting part may be installed on a surface of the first wiring.

Thus, the first wiring is arranged on the opposing surface, whereby the light-emitting part is mounted on the first wiring using, e.g., a bump or another connecting member. The light-emitting part is capable of emitting light using electrical power fed from the first wiring.

According to a third aspect of the invention, light emitted by the light-emitting part may include a first light directed at the detection site and a second light directed in a direction other than that of the detection site; and the biological information detector may further include a first reflecting part for reflecting the second light towards the detection site.

Thus, the second light, which does not directly reach the detection site of the test subject, reaches the detection site via the first reflecting part. Therefore, the amount of light reaching the detection site increases, and the detection accuracy of the biological information detector increases further.

According to a fourth aspect of the invention, the first reflecting part may be secured to the opposing surface.

The distance between the first reflecting part and the detection site can thus be reduced. Therefore, in addition to the amount of the first light reaching the detection site, the amount of the second light reaching the detection site also increases, and the detection accuracy of the biological information detector increases.

According to a fifth aspect of the invention, the first reflecting part may be secured to the opposing surface with an insulating member interposed therebetween.

The insulating member is capable of protecting the opposing surface in an instance in which, e.g., the first wiring for the light-emitting part is arranged on the opposing surface, or in similar instances.

According to a sixth aspect of the invention, the biological information detector may further include a substrate formed from a material that is transparent with respect to the wavelength of light emitted by the light-emitting part, the substrate having a first surface and a second surface disposed opposite the first surface; wherein a second wiring for the light-emitting part may be arranged on the second surface; and the first wiring may be electrically connected to the second wiring with an electroconductive member interposed therebetween.

The first wiring is thus connected to the second wiring. Electrical power can be fed to the light-emitting part via the second wiring, the electroconductive member, and the first wiring.

According to a seventh aspect of the invention,
the biological information detector may further include a second reflecting part for causing light having biological information from the detection site to be reflected and guided towards the light-receiving part; wherein
the light-receiving part may be arranged on the first surface; and
the substrate may be arranged between the second reflecting part and the contact part.

Thus, the substrate is arranged between the second reflecting part and the protecting part. Therefore, even in an instance in which the light-receiving part is arranged on the substrate, there is no need to separately provide a mechanism for supporting the substrate itself, and the number of components is smaller. Also, since the substrate is formed from a material that is transparent with respect to the emission wavelength, the substrate can be disposed on a light path from the light-emitting part to the light-receiving part, and there is no need to accommodate the substrate at a position away from the light path, such as within the reflecting part. A biological information detector that can be readily assembled can thus be provided.

An eighth aspect of the invention relates to a biological information measuring device, characterized in including the biological information detector described above and a biological information measuring part for measuring the biological information from a light reception signal generated in the light-receiving part, wherein the biological information is a pulse rate.

According to the eighth aspect of the invention, the biological information detector with increased detection accuracy can be used to increase the measurement accuracy of the biological information measuring device. Also, the biological information detector can be applied to a pulse rate monitor.

### BRIEF DESCRIPTION OF THE DRAWINGS

FIGS. 1A and 1B are examples of a configuration of the biological information detector according to a present embodiment;

FIGS. 2A, 2B, and 2C are plan views of the biological information detector shown in FIG. 1B;

FIGS. 3A and 3B are comparative examples corresponding to the configuration examples shown in FIG. 1;

FIGS. 4A, 4B, and 4C are plan views of the biological information detector shown in FIG. 3B;

FIGS. 5A and 5B are another example of a configuration of the biological information detector according to the present embodiment;

FIGS. 6A and 6B are schematic diagrams showing a wiring for the light-emitting part;

FIG. 7 is an example of intensity characteristics of light emitted by the light-emitting part;

FIG. 8 is an example of transmission characteristics of light passing through the contact part;

FIG. 9 is an example of the transmission characteristics of light passing through the substrate coated with a light-transmitting film;

FIGS. 10A, 10B, and 10C are examples of a configuration of the first reflecting part;

FIGS. 11A and 11B are examples of the outer appearance of the first reflecting part and the light-emitting part;

FIGS. 12A. 12B, and 12C are examples of the outer appearance of a biological information measuring device including the biological information detector; and

FIG. 13 is an example of a configuration of the biological information measuring device.

### DETAILED DESCRIPTION OF EXEMPLARY EMBODIMENTS

A description shall now be given for the present embodiment. The present embodiment described below is not intended to unduly limit the scope of the claims of the present embodiment. Not every configuration described in the present embodiment is necessarily an indispensable constituent feature of the invention.

### 1. Biological information detector

### 1.1 First configuration example

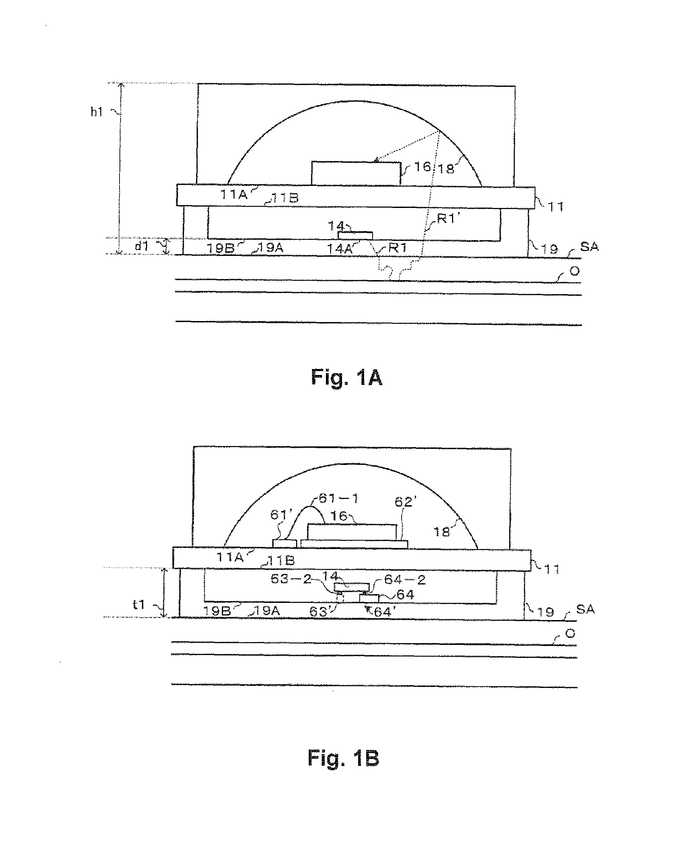

FIGS. 1A and 1B are an example of a configuration of the biological information detector according to a present embodiment. In FIGS. 1A and 1B, the dimensions of each of the members are not intended to accurately represent actual dimensions. Specifically, in FIGS. 1A and 1B, dimensions of each of the members have been expanded or reduced in order to facilitate understanding of the descriptions given below. Similarly, drawings other than FIGS. 1A and 1B are not intended to necessarily represent actual dimensions.

As shown in FIGS. 1A and 1B, a biological information detector includes a light-emitting part 14, a light-receiving part 16, and a contact part 19. The light-emitting part 14 emits light R1 directed at a detection site O of a test subject (e.g., a user). The light-receiving part 16 receives light R1' having biological information (i.e., the reflected light), produced by light R1 emitted by the light-emitting part 14 reflecting at the detection site O. The contact part 19 has a surface 19A that makes contact with the test subject, and an opposing surface 19B disposed opposite the contact surface 19A. The contact part 19 is formed from a material that is transparent with respect to a wavelength of light R1 emitted by the light-emitting part 14 (e.g., glass). The contact part 19 is capable of protecting the light-emitting part 14.

As can be seen in FIG. 1A, the light-emitting part 14 is installed on the opposing surface 19I3. Wiring for the light-emitting part 14 and wiring for the light-receiving part 1.6 are not shown in the example shown in FIG. 1A, but can be represented as shown, e.g., in FIG. 1B. The example shown in FIG. 1B shows a cross-section view along a cut plane. In reality, wiring other than that shown in the example of FIG. 1B is also present. In the example shown in FIG. 1B, a connecting pad 63' and a bump 63-2 that are not, in reality, present in the cut plane, are represented by a dotted line and a white circle. In the example shown in FIG. 1B. a part of a wiring 64 for the light-emitting part 14 is shown. The wiring 64 has a pad 64' for providing a connection with the light-emitting part 14. The pad 64' for providing a connection with the light-emitting part 14 (or in a broader sense, a first wiring for the light-emitting part 14) is arranged on the opposing surface 19B, and the light-emitting part 14 is installed on a surface of the pad 64' for providing a connection with the light-emitting part 14. In the example shown in FIG. 1B, the light-emitting part 14 is, e.g., mounted on the surface of the connecting pad 64' (or in a broader sense, the opposing surface 19B of the contact part 19) using, e.g., a bump 64-2 or another connecting member.

Since the light-emitting part 14 is installed on the opposing surface 19B, the distance between the light-emitting part 14 and the detection site O of the test subject (e.g., a user) can be reduced. Therefore, the amount of light reaching the detection site O increases, and the detection accuracy (i.e., signal-to-noise ratio) of the biological information detector increases. Meanwhile, according to Patent Citation 1, the light-emitting part 11 and the light-receiving part 12 are arranged, with the substrate 15, in the interior of the reflecting part 131; and the interior of the reflecting part 131 is filled with the transparent material 142. According to a configuration of such description, a predetermined distance is present between the light-emitting part 11 and the detection site, and the detection accuracy of the biological information detector is poor.

In the example shown in FIG. 1B, the connecting pad 64' is connected to, e.g., an anode of the light-emitting part 14 via the bump 64-2 (e.g., a gold bump, a solder bump etc.). In the example shown in FIG. 1B, the connecting pad 63' shown by a dotted line is connected, e.g., to a cathode of the light-emitting part 14 via the bump 63-2 shown by a white circle. In the example shown in FIG. 1B, a part of a wiring for the light-receiving part 16 is shown, and a pad 61' for providing a connection to the light-receiving part 16 is shown. The connecting pad 61' is connected, e.g., to an anode of the light-receiving part 16 via a bonding wire 61-1. In the example shown in FIG. 1B, a connecting part 62' in contact with, e.g., a cathode of the light-receiving part 16 is also shown as a part of a wiring for the light-receiving part 16. The connecting part 62' is directly connected to the cathode of the light-receiving part 16 via, e.g., an adhesive (not shown). A silver paste, for example, can be used as an electroconductive adhesive (or in a broader sense, a connecting member).

As shown in FIGS. 1A and 1B, the biological information detector may also include a reflecting part 18. The biological information detector may also be modified so that the biological information detector is configured so as not to include the reflecting part 18 as shown in FIGS. 1A and 1B. The reflecting part 18 reflects the light R1' having biological information (i.e., the reflected light). The reflecting part 18 may have a reflecting surface on a dome surface provided on a light path between the light-emitting part 14 and the light-receiving part 16.

As shown in FIGS. 1A and 1B, the biological information detector may also include a substrate 11. The substrate 11 has a first surface 11A and a second surface 11B disposed opposite the first surface 11A, and is formed from a material that is transparent with respect to the wavelength of light R1 emitted by the light-emitting part 14 (e.g., polyimide). The biological information detector may be modified so that the biological information detector is configured so as to not include the substrate 11 as shown in FIGS. 1A and 1B. In the example shown in FIGS. 1A and 1B, the substrate 11 is arranged between the reflecting part 18 and the contact part 19, and the light-receiving part 16 is placed on the substrate 11 on a side towards the reflecting part 18 (or in a narrower sense, on the first surface 11A of the substrate 11).

The substrate 11 is arranged between the reflecting part 18 and the contact part 19. Therefore, even when the light-receiving part 16 is arranged on the substrate 11, there is no need to separately provide a mechanism for supporting the substrate 11 itself, an the number of components is smaller. Also, since the substrate 11 is formed from a material that is transparent with respect to the emission wavelength, the substrate 11 can be disposed on a light path from the light-emitting part 14 to the light-receiving part 16, and there is no need to accommodate the substrate 11 at a position away from the light path, such as within the reflecting part 18. A biological information detector that can be readily assembled can thus be provided. Also, the reflecting part 18 is capable of increasing the amount of light reaching the light-receiving part 16 or the detection site O, and the detection accuracy (i.e., signal-to-noise ratio) of the biological information detector increases.

In Patent Citation 1, it is necessary to install the light-emitting part 11, the light-receiving part 12, the substrate 15, and the transparent material 142 in the interior of the reflecting part 131. Therefore, a small optical probe 1 cannot be assembled with ease. Also, according to paragraph [0048] in Patent Citation 1, the substrate 15 is formed so that an interior-side of the reflecting part 131 is a diffuse reflection surface. In other words, the substrate 15 in Patent Citation 1 is not required to be formed from a transparent material.

In the examples shown in FIGS. 1A and 1B, the detection site O (e.g., a blood vessel) is within the test subject. The first light R1 travels into the test subject, and diffuses or scatters at the epidermis, the dermis, and the subcutaneous tissue. Then, the first light R1 reaches the detection site O, and is reflected at the detection site O. The reflected light R1' reflected at the detection site O diffuses or scatters at the subcutaneous tissue, the dermis, and the epidermis. The light R1 is partially absorbed at the blood vessel. Therefore, due to an effect of a pulse, the rate of absorption at the blood vessel varies, and the amount of the reflected light R1' reflected at the detection site O also varies. Biological information (e.g., pulse rate) is thus reflected in the reflected light R1' reflected at the detection site O.

The thickness of the substrate 11 is e.g., 10 µm to 1000 µm. Wiring for the light-emitting part 14 and wiring for the light-receiving part 16 may be formed on the substrate 11. The substrate 11 is, e.g., a printed circuit board; however, a printed circuit board is not generally formed from a transparent material, as with the substrate 15 of Patent Citation 1. Specifically, the inventors purposefully used a configuration in which the printed circuit board is formed from a material that is transparent at least with respect to the emission wavelength of the light-emitting part 14. The thickness of the protecting part 19 is, e.g., 1 µm to 3000 µm.

Examples of configurations of the biological information detector are not limited by that shown in FIGS. 1A and 1B, and the shape, or a similar attribute, of a part of the example of configuration (e.g., the light-receiving part 16) may be modified. The biological information may also be blood oxygen saturation level, body temperature, heart rate, or a similar variable; and the detection site O may be positioned at a surface SA of the test subject. In the example shown in FIGS. 1A and 1B, the first light R1 is shown by a single line; however, in reality, the light-emitting part 14 emits many light beams in a variety of directions

The light-emitting part 14 is, for example, an LED. The light emitted by the LED has a maximum intensity (or in a broader sense, a peak intensity) within a wavelength range of, e.g., 425 nm to 625 nm, and is, e.g., green in color. The thickness of the light-emitting part 14 is, e.g., 20 nm to 1000 µm. The light-receiving part 16 is, e.g., a photodiode, and can generally be formed by a silicon photodiode. The thickness of the light-receiving part 16 is, e.g., 20 µm to 1000 µm. The silicon photodiode has a maximum sensitivity (or in a broader sense, a peak sensitivity) for received light having a wavelength within a range of, e.g., 800 nm to 1000 nm. Ideally, the light-receiving part 16 is formed by a gallium arsenide phosphide photodiode, and the gallium arsenide phosphide photodiode has a maximum sensitivity (or in a broader sense, a peak sensitivity) for received light having a wavelength within a range of, e.g., 550 nm to 650 nm. Since biological substances (water or hemoglobin) readily allow transmission of infrared light within a wavelength range of 700 nm to 1100 nm, the light-receiving part 16 formed by the gallium arsenide phosphide photodiode is more capable of reducing noise components arising from external light than the light-receiving part 16 formed by the silicon photodiode.

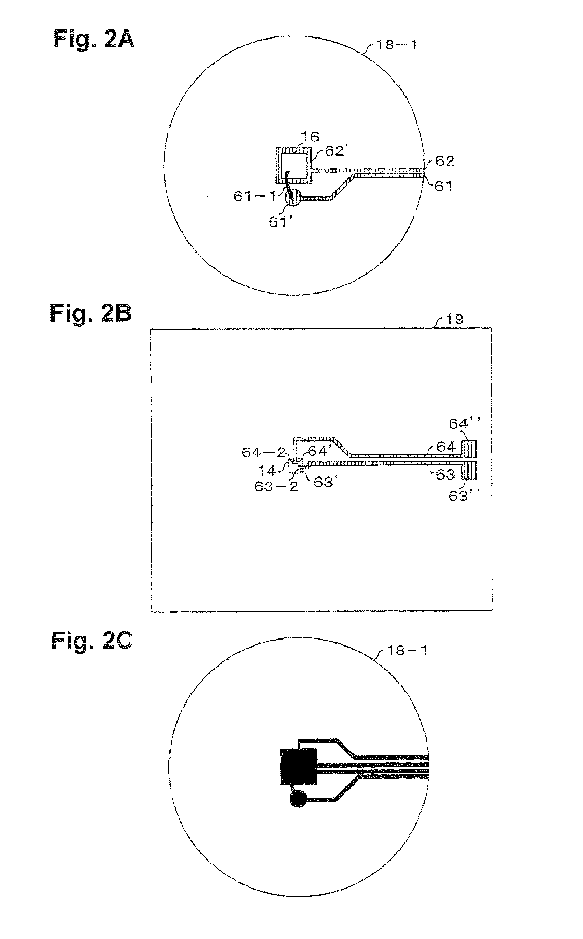

FIGS. 2A, 2B, and 2C are plan views of the biological information detector shown in FIG. 1B. FIG. 2A, corresponds to a plan view of a side towards the light-receiving part 16, FIG. 2B corresponds to a plan view of a side towards the light-emitting part 14, and FIG. 2C corresponds to a light-blocking region including the light-receiving part 16 and the light-emitting part 14. FIGS. 2A and 2C show only a region of irradiation in which the light R1' having biological information (i.e., the reflected light) travels to the substrate 11. The irradiation region may be defined, e.g., by a boundary 18-1 between the reflecting surface of the reflecting part 18 (i.e., the dome surface in each of the examples shown in FIGS. 1A and 1B) and the substrate 11. The boundary 18-1 has, for example, a circular profile.

As shown in FIG. 2A, in plan view (when, e.g., viewed from a side towards the light-receiving part 16 in FIG. 1B), a wiring 61 that connects to an anode (or in a broader sense, an electrode) of the light-receiving part 16 is formed on the substrate 11. A wiring 62 that connects to a cathode (or in a broader sense, an electrode) of the light-receiving part 16 is also formed on the substrate 11. In the example shown in FIG. 2A, the wiring 61 has a connecting pad 61' that connects to the light-receiving part 16, and the bonding wire 61-1. The connecting pad 61' of the wiring 61 is connected to the anode of the light-receiving part 16 via the bonding wire 61-1. In the example shown in FIG. 2A, the wiring 62 has a connecting part 62' in contact with the cathode of the light-receiving part 16

As shown in FIG. 2B, in plan view (when, e.g., viewed from a side towards the light-emitting part 14 in FIG. 1B), a wiring 63 that connects to a cathode (or in a broader sense, an electrode) of the light-emitting part 14 is formed on the contact part 19 (or in a narrower sense, the opposing surface 19B). A wiring 64 that connects to an anode (or in a broader sense, an electrode) of the light-emitting part 14 is also formed on the contact part 19 (or in a narrower sense, the opposing surface 19B). In the example shown in FIG. 2B, the wiring 63 has the connecting pad 63' that connects to the light-emitting part 14, and the bump 63-2. The connecting pad 63' of the wiring 63 is connected to the cathode of the light-emitting part 14 via the bump 63-2. The wiring 63 may include a connecting pad 63". In the example shown in FIG. 2B, the wiring 64 has the connecting pad 64' that connects to the light-emitting part 14, and the bump 64-2. The connecting pad 64' of the wiring 64 is connected to the anode of the light-emitting part 14 via the bump 64-2. The wiring 64 may include a connecting pad 64".

The configuration of the wiring 63 and the wiring 64 to the light-emitting part 14 and the wiring 61 and the wiring 62 to the light-receiving part 16 are not limited by the examples shown in FIGS. 2A and 2B. For example, the shape of the connecting pad 61' of the wiring 61 may, instead of being circular as shown in FIG. 2A, be, e.g., square, elliptical, polygonal, or describing another shape. The shape of the connecting pads 63', 63" of the wiring 63 may, instead of being square as shown in FIG. 2B, also be, e.g., circular, elliptical, polygonal, or describing another shape. Also, although in the example shown in FIG. 2A, the light-receiving part 16 has the cathode on a bottom surface, the light-receiving part 16 may have the cathode on a front surface in a similar manner to the anode.

As shown, for example, in FIG. 1A, in an instance in which the light R1' having the biological information, (i.e., the reflected light) travels to the substrate 11, the light R1' having the biological information (i.e., the reflected light) reaches the opposing surface 19B of the contact part 19. In an instance in which the wiring 63 and the wiring 64 to the light-emitting part 14 are present as shown in FIG. 2B, at least the wiring 63 and the wiring 64 block or reflect the light R1' having the biological information (i.e., the reflected light) and form a light-blocking region. Also, even in an instance where the light R1' having the biological information (i.e., the reflected light) enters an interior of the substrate 11, in an instance where the wiring 61 and the wiring 62 to the light-receiving part 16 are present as shown in FIG. 2A, at least the wiring 61 and the wiring 62 inhibit the light R1' having the biological information (i.e., the reflected light) from leaving the interior towards an exterior of the substrate 11. The light-blocking region of the contact part 19 and the substrate 11, where the wiring 61, the wiring 62, the wiring 63, and the wiring 64 are positioned, thus inhibit the light R1' having the biological information (i.e., the reflected light) from reaching the reflecting part 18. Specifically, the light R1' having the biological information (i.e., the reflected light) is capable of passing through a region of the substrate 11 excluding the light-blocking region of the contact part 19 and the substrate 11.

FIG. 2C shows a light-blocking region within the irradiation region. The light-blocking region is shown in black in the example shown in FIG. 2C. As shown in FIG. 2C, the light-blocking region can be defined, with respect to the plan view, by the wiring 61 (including the connecting pad 61' and the bonding wire 61-1) and the wiring 62 (including the connecting part 62') shown in FIG. 2A, and the wiring 63 (including the connecting pad 63' and the bump 63-2) and the wiring 64 (including the connecting pad 64' and the bump 64-2) shown in FIG. 2B.

### 1.2 Comparative example

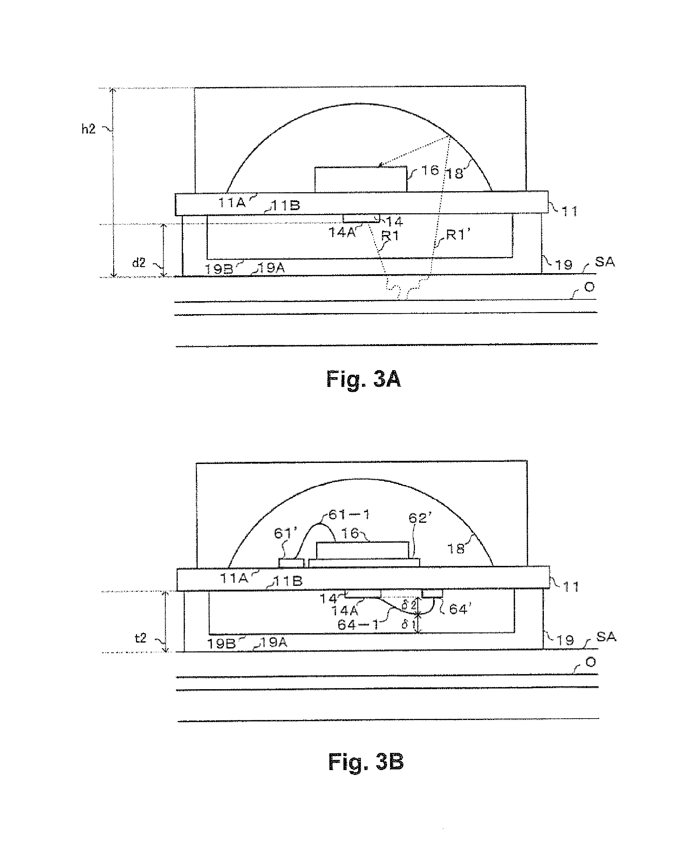

FIGS. 3A and 3B are a comparative example corresponding to the configuration examples shown in FIG. 1. Structures that are identical to those in the examples described above are affixed with the same numerals, and a description of the structures is not provided. The example shown in FIGS. 3A and 3B is a comparative example but have a novel configuration. In the example shown in FIG. 3A, the light-emitting part 14 is arranged on the second surface 11B of the substrate 11. Specifically, as shown in FIG. 3B, the pad 64' for providing a connection with the light-emitting part 14 (or in a broader sense, the first wiring for the light-emitting part 14) is arranged on the second surface 11B, and the connecting pad 64' is connected to, e.g., the anode of the light-emitting part 14 via a bonding wire 64-1.

In the example shown in FIG. 3A, the distance between a first light-emitting surface 14A that faces the detection site O and emits a first light R1, and the surface SA of the test subject, is represented by d2. In FIG. 1A, the distance between the first light-emitting surface 14A and the surface SA of the test subject is represented by d1. In the example shown in FIG. 1A, since the light-emitting part 14 is installed on the opposing surface 19B, d1 is smaller than d2. Since the distance between the light-emitting part 14 and the detection site O is therefore shorter, the amount of light reaching the detection site O increases, and the detection accuracy (i.e., the signal-to-noise ratio) of the biological information detector increases.

In an instance in which the light-emitting part 14 is arranged on the second surface 11B of the substrate 11 as shown in FIG. 3B, a bonding wire 64-1 becomes necessary. The bonding wire 64-1 is arranged between the connecting pad 64' and the anode of the light-emitting part 14. As shown in FIG. 3B, the bonding wire 64-1. describes an arc, and the height or the depth of the bonding wire 64-1 (i.e., the arc) is represented by δ2. δ2 is, e.g., 120 µm. A gap which is represented by δ1 in FIG. 3B is provided so that the contact part 19 does not damage the bonding wire 64-1. δ1 is, e.g., 300 µm. When error during manufacture of the bonding wire 64-1 and flexure of the substrate 11 is taken into account, δ1 must not be zero.

Therefore, the thickness t2 of the contact part 19 shown in FIG. 3B is larger than the thickness t1 of the contact part 19 shown in FIG. 3B. The height h2 of the biological information detector shown in FIG. 3A is thereby larger than the height h1 of the biological information detector shown in FIG. 1A. Specifically, in the example shown in FIGS. 1A and 1B, the biological information detector can be made smaller.

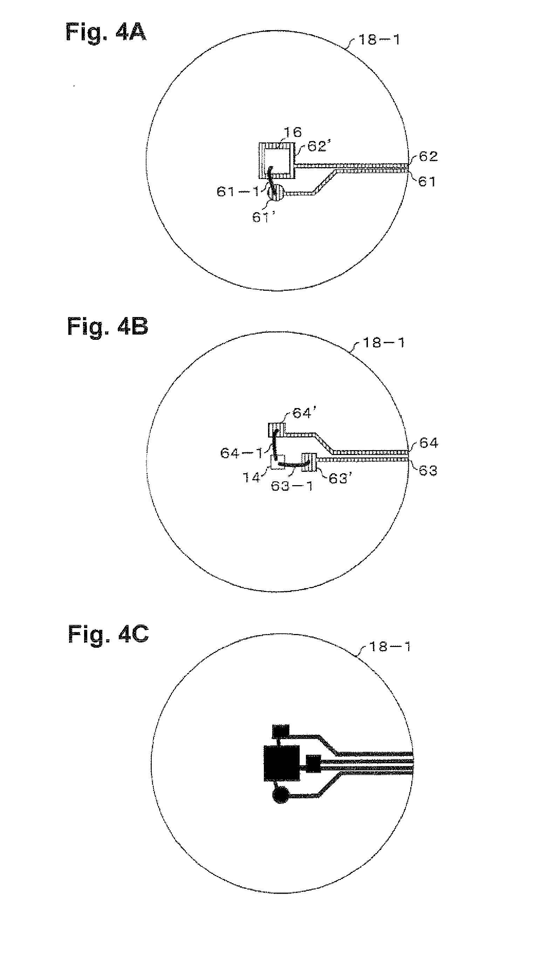

FIGS. 4A, 4B, and 4C are plan views of the biological information detector shown in FIG. 3B. FIG. 4A corresponds to a plan view of a side towards the light-receiving part 16. FIG. 4B corresponds to a plan view of a side towards the light-emitting part 14, and FIG. 4C corresponds to a light-blocking region including the light-receiving part 16 and the light-emitting part 14. Structures that are identical to those in the examples described above are affixed with the same numerals, and a description of the structures is not provided. FIG. 4A matches FIG. 2A. However, in the example of FIG. 4B, the wiring 63 has a pad 63' for providing a connection with the light-emitting part 14, and a bonding wire 63-1. The connecting pad 63' of the wiring 63 is connected to the cathode of the light-emitting part 14 via the bonding wire 63-1. In the example of FIG. 4B, the wiring 64 has a pad 64' for providing a connection with the light-emitting part 14, and a bonding wire 64-1. The connecting pad 64' of the wiring 64 is connected to the anode of the light-emitting part 14 via the bonding wire 64-1.

The connecting pad 63' and the connecting pad 64' of FIG. 4B are respectively connected to the bonding wire 63-1 and the bonding wire 64-1, the connection being established externally with respect to the light-emitting part 14. Therefore, the light-blocking region shown in FIG. 2C is smaller than the light-blocking region shown in FIG. 4C. Accordingly, in the example shown in FIG. 2C, the light R1' having biological information (i.e., the reflected light) can readily reach the light-emitting part 14, and the detection accuracy (i.e., the signal-to-noise ratio) of the biological information detector increases.

### 1.3 Second configuration example

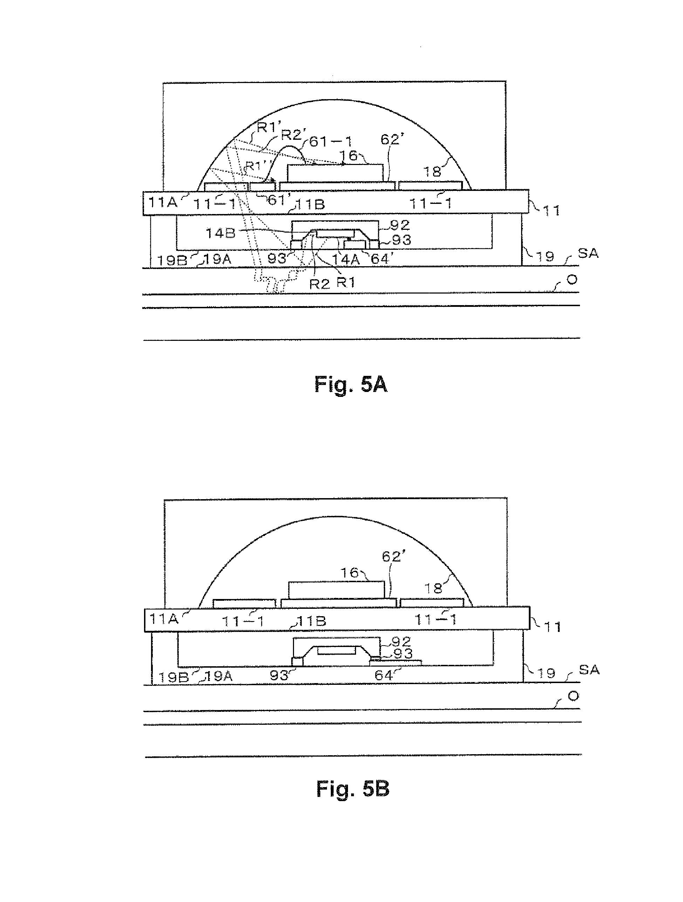

FIGS. 5A and 5B show another example of a configuration of the biological information detector according to the present embodiment. FIG. 5A is a cross-section view corresponding to the same cut plane as that shown in FIG. 1B, and FIG. 5B is a cross-section view corresponding to another cut surface. Structures that are identical to those in the examples described above are affixed with the same numerals, and a description of the structures is not provided. As shown in FIGS. 5A and 5B, the biological information detector may further include a reflecting part 92. In an instance in which the reflecting part 92 is referred to as a first reflecting part, the reflecting part 18 may be referred to as a second reflecting part. In the example shown in FIGS. 5A and 5B, the first reflecting part 92 is secured to the opposing surface 19B of the contact part 19. The first reflecting part 92 can be secured using, e.g., an adhesive 93.

As shown in FIG. 5A, the light-emitting part 14 emits a first light R1 directed at the detection site O of the test subject (e.g., a user) and a second light R2 directed in a direction other than that of the detection site O (i.e., directed at the first reflecting part 92). The first reflecting part 92 causes the second light R2 to be reflected and guided towards the detection site O. The light-receiving part 16 receives lights R1' and R2' having biological information (i.e., reflected light; valid light), which are, respectively, first light R1 and second light R2 reflected at the detection site O. The second reflecting part 18 causes lights R1' and R2' having biological information (i.e., reflected light) from the detection site O to be reflected and guided towards the light-receiving part 16. Due to the presence of the first reflecting part 92, second light R2, which does not directly reach the detection site O of the test subject (i.e., the user), also reaches the detection site O. Specifically, the amount of light reaching the detection site O via the first reflecting part 92 increases, and the efficiency of the light-emitting part 14 increases. Therefore, the detection accuracy (i.e., the signal-to-noise ratio) of the biological information detector increases.

In Patent Citation 1, there is disclosed a structure corresponding to the second reflecting part 18 (i.e., the reflecting part 131 in FIG. 16 of Patent Citation 1). Specifically, the light-receiving part 12 in FIG. 16 of Patent Citation 1 receives light reflected at the detection site via the reflecting part 131. However, in Patent Citation 1 a structure corresponding to the first reflecting part 92 is not disclosed. In other words, at the time of application, those skilled in the art have not identified an issue of increasing the efficiency of the light-emitting part 11 in FIG. 16 in Patent Citation 1.

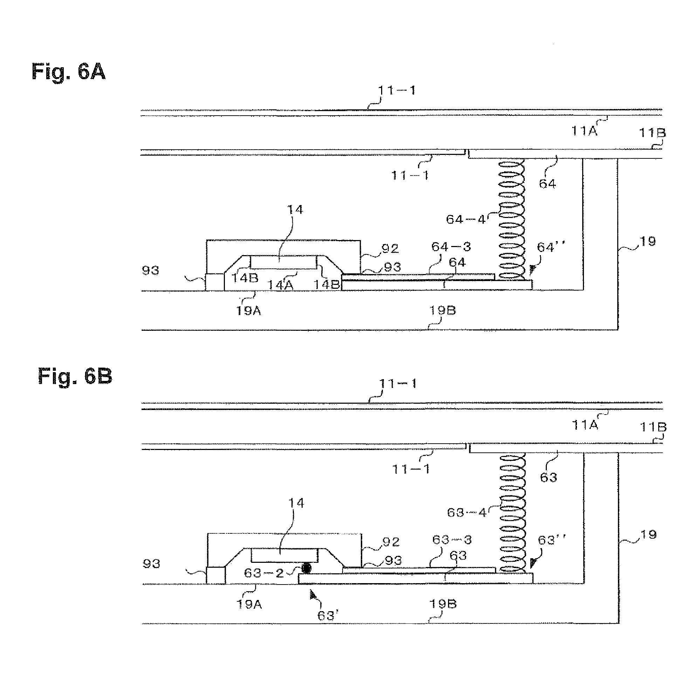

As can be seen in FIGS. 5A and 5B, a light-transmitting film 11-1 can be formed on the first surface (e.g., a front surface) of the substrate 11. The light-transmitting film 11-1 may also be formed on the second surface (e.g., a reverse surface) that is opposite the first surface (see FIGS. 6A and 6B). The light-transmitting film 11-1 can be formed on a light-transmitting region (i.e., an irradiation region) of the substrate 11 excluding the light-blocking region of the substrate 11 where, e.g., the connecting pad 61', the connecting part 62', and similar components are arranged. The light-transmitting film 11-1 can be formed from, e.g., a solder resist (or, in a broader sense, a resist).

Each of FIGS. 6A and 6B is a schematic diagram showing a wiring for the light-emitting part 14. FIG. 6A corresponds to FIG. 5B, and FIG. 6B is a cross-section view corresponding to a cut plane that is different from that shown in FIG. 6A. Structures that are identical to those in the examples described above are affixed with the same numerals, and a description of the structures is not provided. As shown in FIG. 6A, in an instance in which a wiring 64 (i.e., a second wiring for the light-emitting part 14) is arranged on the second surface 11B of the substrate 11, the wiring 64 arranged on the opposing surface 19B of the contact part 19 (i.e., a first wiring for the light-emitting part 14) is electrically connected to the wiring 64 arranged on the second surface 11B of the substrate 11 (i.e., the second wiring for the light-emitting part 14) via an electroconductive member. In the example shown in FIG. 6A, the electroconductive member is, e.g., a spring 64-4. Using, e.g., gold plating on the spring makes the spring 64-4 electrically conductive. The electroconductive member may also be, e.g., an electroconductive rubber. In the example shown in FIG. 6B, the wiring 63 arranged on the opposing surface 19B of the contact part 19 (i.e., a first wiring for the light-emitting part 14) is electrically connected to the wiring 64 arranged on the second surface 11B of the substrate 11 (i.e., a second wiring for the light-emitting part 14) via an electroconductive member (e.g., a spring 63-4, an electroconductive rubber, etc.). In the example shown in FIG. 6B, the light-emitting part 14 is installed on a surface of the wiring 63 (i.e., the first wiring for the light-emitting part 14) via the bump 63-2.

In an instance in which the first reflecting part 92 is secured to the wiring 64 as shown in FIG. 5B, the thickness of the adhesive 93 may decrease. Therefore, in order to protect the wiring 64 (i.e., the wiring for the light-emitting part 14) (or in a broader sense, the opposing surface 19B), an insulating member 64-3 may be provided on the wiring 64 as shown in FIG. 6A. Also, in order to protect the wiring 63 (i.e., the wiring for the light-emitting part 14) (or in a broader sense, the opposing surface 19B), an insulating member 63-3 may be provided on the wiring 63 as shown in FIG. 6B. The first reflecting part 92 is thus secured on the opposing surface 19B via the insulating members 63-3, 64-3. As with the light-transmitting film 11-1, the insulating members 63-3, 64-3 can be formed from, e.g., a solder resist (or, in a broader sense, a resist).

The first surface 11A and the second surface 11B of the substrate 11 shown in FIGS. 5A, 5B, 6A, and 6B may be processed so as to form a rough surface so that the wiring 63 and the wiring 64 on the substrate 11 do not peel off. Therefore, the light-transmitting film 11-1 is formed on at least one of the first surface 11A and the second surface 11B, whereby the roughness on the surface of the substrate 11 is filled with the light-transmitting film, and the smoothness of the entire substrate 11 is increased. Specifically, the light-transmitting film 11-1 on the substrate 11 is smooth, and can therefore reduce dispersion of light on the roughness on the surface of the substrate 11 during transmission of the light through the substrate 11. Specifically, the presence of the light-transmitting film 11-1 increases the transmittance of the substrate 11. Therefore, the amount of light reaching the detection site O increases, and the detection accuracy of the biological information detector increases further.

The refractive index of the light-transmitting film 11-1 is preferably between the refractive index of air and the refractive index of the substrate 11. Further preferably, the refractive index of the light-transmitting film 11-1 is preferably closer to the refractive index of the substrate 11 than the refractive index of air. In such an instance, it is possible to reduce reflection of light on an interface.

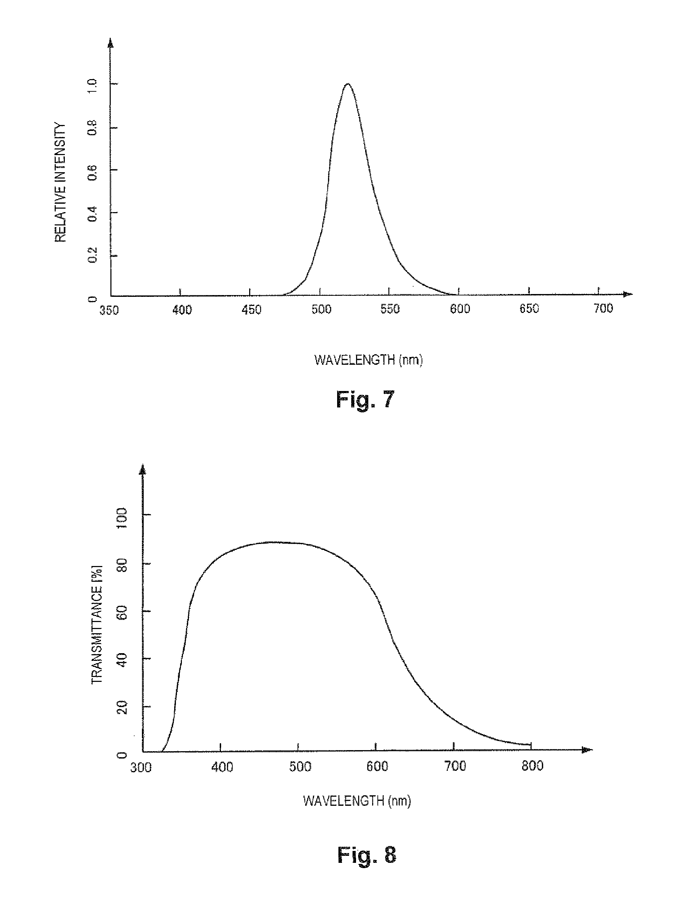

FIG. 7 shows an example of intensity characteristics of the light emitted by the light-emitting part 14. In the example shown in FIG. 7, the intensity is at a maximum for light having a wavelength of 520 nm, and the intensity of light having other wavelengths is normalized with respect thereto. Also, in the example shown in FIG. 7, the wavelengths of light emitted by the light-emitting part 14 are within a range of 470 nm to 600 nm.

FIG. 8 shows an example of transmission characteristics of light passing through the contact part 19. As shown in FIG. 8, the transmittance at the wavelength of light emitted by the light-emitting part 14 where the intensity is at the maximum shown, e.g., in FIG. 7 (i.e., 520 nm) is 50% or above. As for an example of transmission characteristics of light passing through the substrate 11 itself, although not shown, transmittance of the substrate 11 with respect to the wavelength of 520 nm can be set to, e.g., 50% or above, as with the transmission characteristics shown in FIG. 8. The contact part 19 and the substrate 11 can be formed from a material that is transparent with respect to the wavelength of light R1 emitted by the light-emitting part 14.

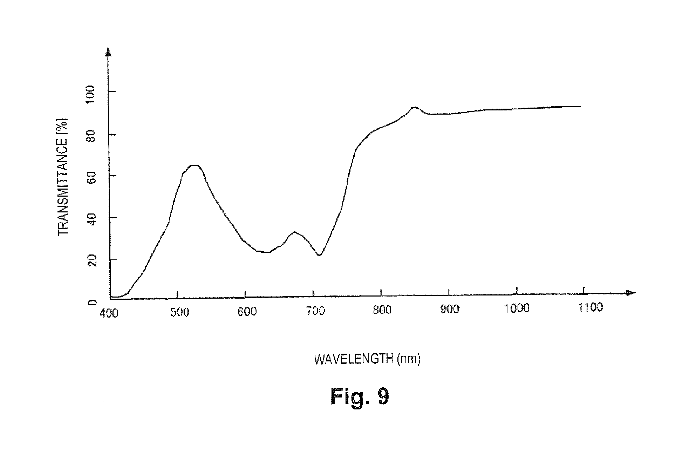

FIG. 9 is an example of transmission characteristics of light passing through the substrate 11 coated with a light-transmitting film 11-1. In the example shown in FIG. 9, transmittance is calculated using the intensity of light before being transmitted through the substrate 11 and the intensity of light after being transmitted through the substrate 11. In the example shown in FIG. 9, in a region of wavelength equal to or less than 700 nm, which is the lower limit of the biological window, the transmittance is at a maximum for light having a wavelength of 525 nm. Or, in the example shown in FIG. 9, in the region of wavelength equal to or less than 700 nm, which is the lower limit of the biological window, the maximum transmittance of light passing through the light-transmitting film 11-1 falls within a range of ±10% of the maximum intensity of the light with a certain wavelength generated by the light-emitting part 14 in FIG. 7, for example. It is preferable that the light-transmitting film 11-1 thus selectively transmit light generated by the light-emitting part 14 (e.g., the first light R1 in FIG. 1A (or in a narrower sense, the reflected light R1' produced by the first light R1 being reflected)). The presence of the light-transmitting film 11-1 makes it possible to enhance the smoothness of the substrate 11 and prevent, to a certain extent, a decrease in efficiency of the light-emitting part 14 or the light-receiving part 16. In an instance in which transmittance has a maximum value (or in a broader sense, a peak value) within, e.g., a visible light region for light having a wavelength of 525 nm, as shown in the example in FIG. 9, the light-transmitting film 11-1 is, e.g., green.

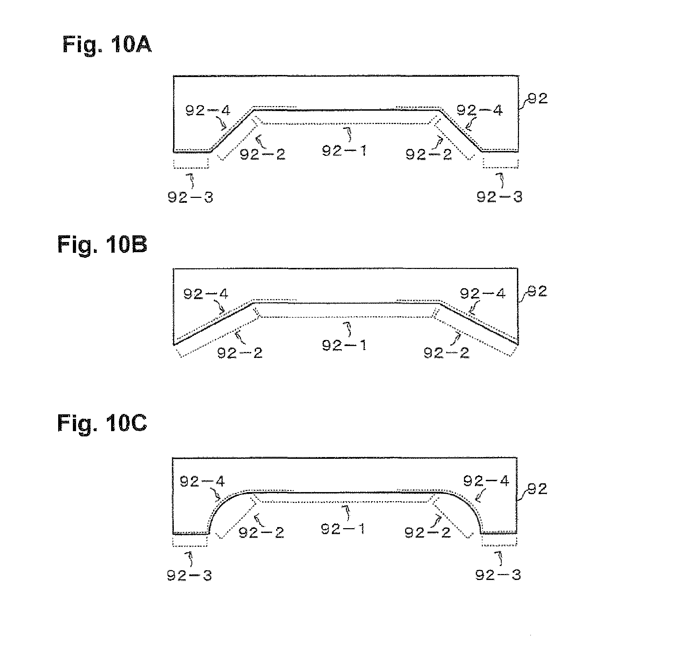

FIGS. 10A, 10B, and 10C are examples of a configuration of the first reflecting part 92 shown in FIGS. 5A, 5B, 6A, and 6B. As shown in FIG. 10A, the first reflecting part 92 may have a support part 92-1 for supporting the light-emitting part 14, and an inner wall surface 92-2 and a top surface 92-3 of a wall part surrounding the second light-emitting surface 14B of the light-emitting part 14. The light-emitting part 14 is not shown in FIGS. 10A through 10C. In the example shown in FIG. 10A, the first reflecting part 92 can reflect the second light R2 on the inner wall surface 92-2 towards the detection site O (see FIG. 5A), the first reflecting part 92 having a first reflecting surface on the inner wall surface 92-2. The thickness of the support part 92-1 is, e.g., 50 µm to 1000 µm, and the thickness of the wall part (i.e., 92-3) is, e.g., 100 µm to 1000 µm.

In the example shown in FIG. 10A, the inner wall surface 92-2 has an inclined surface (92-2) which, with increasing distance in a width direction (i.e., a first direction) from a center of the first reflecting part 92, inclines towards the detection site O in a height direction (i.e., a direction that is perpendicular to the first direction), in cross-section view. The inclined surface (92-2) in FIG. 10A is formed by, in cross-section view, an inclined plane, but may also be a curved surface shown in e.g., FIG. 10C, or a similar inclined surface. The inner wall surface 92-2 may also be formed as a plurality of inclined flat surfaces whose angle of inclination vary from one another, or by a curved surface having a plurality of curvatures. In an instance in which the inner wall surface 92-2 of the first reflecting part 92 has an inclined surface, the inner wall surface 92-2 of the first reflecting part 92 is capable of reflecting the second light R2 towards the detection site O. In other words, the inclined surface on the inner wall surface 92-2 of the first reflecting part 92 can be said to be the first reflecting surface for improving the directivity of the light-emitting part 14. In such an instance, the amount of light reaching the detection site O increases further. The top surface 92-3 shown in FIGS. 10A and 10C may be omitted as shown, e.g., in FIG. 10B. In FIGS. 10A through 10C, a range indicated by label 92-4 function as a mirror surface part.

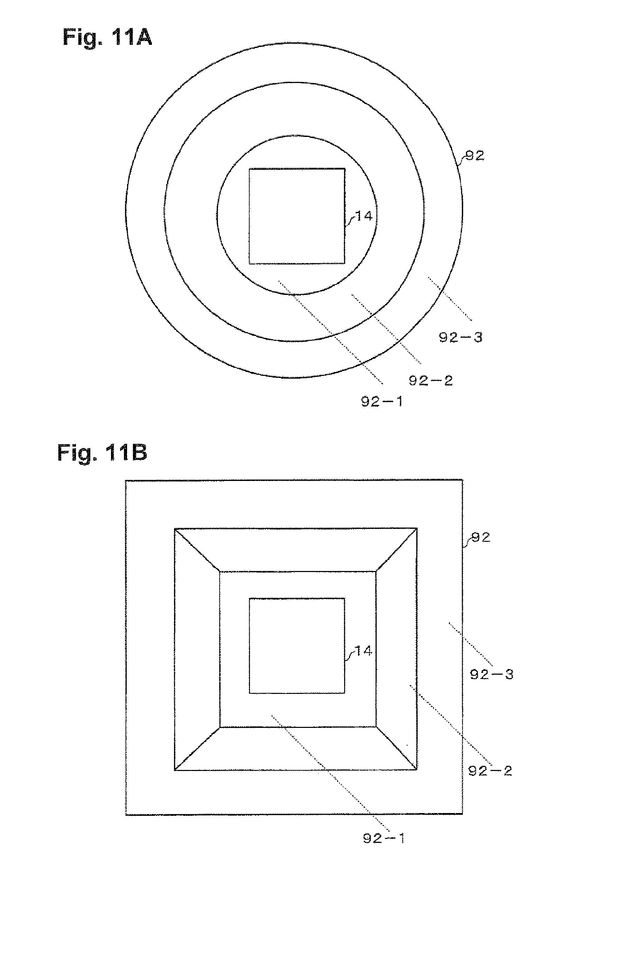

FIGS. 11A and 11B respectively show an example of an outer appearance of the first reflecting part 92 and the light-emitting part 14 of FIGS. 5A, 5B, 6A, and 6B in plan view. In the example shown in FIG. 11A, in plan view (when viewed from, e.g., towards the detection site O shown in FIG. 5A), an outer circumference of the first reflecting part 92 is circular, where the diameter of the circle is, e.g., 200 µm to 11,000 µm. In the example shown in FIG. 11A, the wall part (92-2) of the first reflecting part 92 surrounds the light-emitting part 14 (see FIGS. 5A and 10A). The outer circumference of the first reflecting part 92 may also be a quadrilateral (or specifically, a square) in plan view as shown, e.g., in FIG. 11B. Also, in the examples shown in FIGS. 11A and 11B, in plan view (when viewed from, e.g., towards the detection site O shown in FIG. 5A), the outer circumference of the light-emitting part 14 is a quadrilateral (or specifically, a square), where the length of one side of the square is, e.g., 100 µm to 10,000 µm. The outer circumference of the light-emitting part 14 may also be circular.

The first reflecting part 92 is made of metal whose surface is polished to a mirror finish, and thereby has a reflective structure (or specifically, a mirror reflection structure). The first reflecting part 92 may also be formed from, e.g., a resin whose surface is polished to a mirror finish. Specifically, for example, a base metal forming a base of the first reflecting part 92 is readied, and a surface of the base metal is then, e.g., subjected to plating. Alternatively, a mold of the first reflecting part 92 (not shown) is filled with a thermoplastic resin, molding is performed, and a metal film, for example, is then deposited by vapor deposition on a surface of the mold.

In the examples shown in FIGS. 11A and 11B, in plan view (when viewed from, e.g., towards the detection site O shown in FIG. 5A), a region of the first reflecting part 92 other than that directly supporting the light-emitting part 1.4 (i.e., the inner wall surface 92-2 and the top surface 92-3 of the wall part, and a part of the support part 92-1) is exposed. The exposed region is shown as a mirror surface part 92-4 in FIG. 10A. Although in the example shown in FIG. 10A, a dotted line representing the mirror surface part 92-4 is shown within the first reflecting part 92, the mirror surface part 92-4 is actually formed on a surface of the first reflecting part 92.

In the examples shown in FIGS. 10A, 10B, and 10C, the mirror surface part 92-4 preferably has a high reflectivity. The reflectivity of the mirror surface part 92-4 is, e.g., 80% to 90% or higher. It is possible for the mirror surface part 92-4 to be formed only on the inclined surface of the inner wall surface 92-2. In an instance in which the mirror surface part 92-4 is formed not only on the inclined surface of the inner wall surface 92-2 but also on the support part 92-1, the directivity of the light-emitting part 14 increases further.

In the example shown in FIG. 5A, the second light R2 also travels into the test subject, and the reflected light R2' reflected at the detection site O travels towards the second reflecting part 18. Biological information (i.e., the pulse rate) is also reflected in the reflected light R2' reflected at the detection site O. In the example shown in FIG. 5A, the first light R1 is partially reflected at a surface SA of the test subject (i.e., skin surface). In an instance in which the detection site O is within the test subject, biological information (i.e., the pulse rate) is not reflected in reflected light R1" reflected at the surface SA of the test subject (i.e., directly reflected light).

The second reflecting part 18 is formed from, e.g., a resin whose surface (i.e., a reflecting surface on a side towards the light-receiving part 16) is polished to a mirror finish and thereby has a reflective structure (or specifically, a mirror reflection structure). In other words, the second reflecting part 18 is capable of causing mirror reflection of light without causing diffuse reflection of light. In an instance in which the second reflecting part 18 has a mirror reflection structure, the second reflecting part 18 is also capable of not causing the light R1" produced by reflection of the first light R1 (i.e., the directly reflected light; invalid light) to reflect towards the light-receiving part 16, the reflected light R1" having a reflection angle that is different from that of the light R1' produced by reflection of the first light R1 (see FIG. 5A). In such an instance, the detection accuracy of the biological information detector is further increased. As shown in FIG. 5A, since the light R1' produced by reflection of the first light R1 originates from the detection site O, which is within the test subject, the reflection angle of the light R1' produced by reflection of the first light R1 (i.e., a reflection angle relative to a straight line perpendicular to the surface SA of the test subject) is generally small. Meanwhile, since the light R1" produced by reflection of the first light R1 originates from the surface SA of the test subject, the reflection angle of the light R1" produced by reflection of the first light R1 is generally large.

In FIG. 16 of Patent Citation 1, there is disclosed a reflecting part 131; and according to paragraphs [0046], [0059], and [0077] in Patent Citation 1, the reflecting part 131 has a diffuse reflection structure, and the reflectivity is increased to increase the efficiency of the light-receiving part 12. However, at the time of filing, it had not been recognized by those skilled in the art that in the reflecting part 131 according to Patent Citation 1, directly reflected light (or in a broader sense, noise) is also reflected towards the light-receiving part 12. In other words, the inventors recognized that reducing a noise component arising from the directly reflected light from a light reception signal increases the efficiency of the light-receiving part. Specifically, the inventors recognized that the detection accuracy of the biological information detector is further increased in an instance in which the second reflecting part 18 has a mirror reflection structure.

### 2. Biological information measuring device

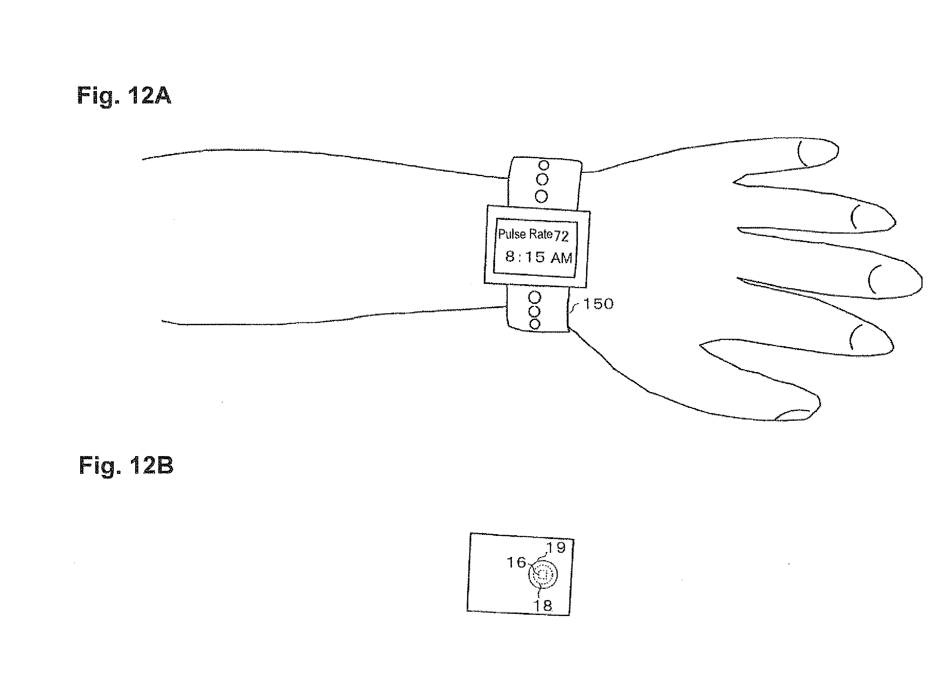

FIGS. 12A and 12B are examples of the outer appearance of a biological information measuring device including the biological information detector such as that shown in FIGS. 1A, 5A, and other drawings. As shown in FIG. 12A, the biological information detector shown, e.g., in FIG. 1 may further include a wristband 150 capable of attaching the biological information detector to an arm (or specifically, a wrist) of the test subject (i.e., the user). In the example shown in FIG. 12A, the biological information is the pulse rate indicated by, e.g., "72." The biological information detector is installed in a wristwatch showing the time (e.g., "8:15 am"). As shown in FIG. 12B, an opening part is provided to a back cover of the wristwatch, and the contact part 19 shown in FIG. 1, for example, is exposed in the opening part. In the example shown in FIG. 12B, the second reflecting part 18 and the light-receiving part 16 are installed in a wristwatch. In the example shown in FIG. 12B, the first reflecting part 92, the light-emitting part 14, the wristband 150, and other components are not shown.

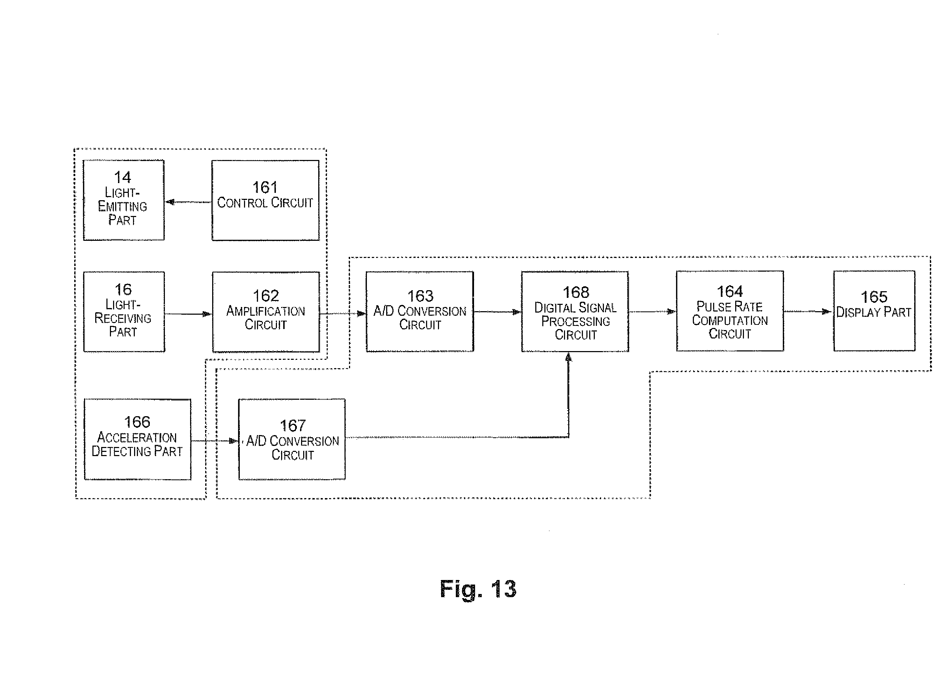

FIG. 13 is an example of a configuration of the biological information measuring device. The biological information measuring device includes the biological information detector as shown, e.g., in FIGS. 1A and 5A, and a biological information measuring part for measuring biological information from a light reception signal generated at the light-receiving part 16 of the biological information detector. As shown in FIG. 13, the biological information detector may have the light-emitting part 14, the light-receiving part 16, and a circuit 161 for controlling the light-emitting part 14. The biological information detector may further have a circuit 162 for amplifying the light reception signal from the light-receiving part 16. The biological information measuring part may have an A/D conversion circuit 163 for performing A/D conversion of the light reception signal from the light-receiving part 16, and a pulse rate computation circuit 164 for calculating the pulse rate. The biological information measuring part may further have a display part 165 for displaying the pulse rate.

The biological information detector may have an acceleration detecting part 166, and the biological information measuring part may further have an A/D conversion circuit 167 for performing A/D conversion of an acceleration signal from the acceleration detecting part 166 and a digital signal processing circuit 168 for processing a digital signal. The configuration of the biological information measuring device is not limited to that shown in FIG. 13. The pulse rate computation circuit 164 in FIG. 13 may be, e.g., an MPU (i.e., a micro processing unit) of an electronic device installed with the biological information detector.

The control circuit 161 in FIG. 13 drives the light-emitting part 14. The control circuit 161 is, e.g., a constant current circuit, delivers a predetermined voltage (e.g., 6 V) to the light-emitting part 14 via a protective resistance, and maintains a current flowing to the light-emitting part 14 at a predetermined value (e.g., 2 mA). The control circuit 161 is capable of driving the light-emitting part 14 in an intermittent manner (e.g., at 128 Hz) in order to reduce consumption current. The control circuit 161 is formed on, e.g., a motherboard, and wiring between the control circuit 161 and the light-emitting part 14 is formed, e.g., on the substrate 11 and the contact part 19 shown in FIGS. 6A and 6B.

The amplification circuit 162 shown in FIG. 13 is capable of removing a DC component from the light reception signal (i.e., an electrical current) generated in the light-receiving part 16, extracting only an AC component, amplifying the AC component, and generating an AC signal. The amplification circuit 162 removes the DC component at or below a predetermined wavelength using, e.g., a high-pass filter, and buffers the AC component using, e.g., an operational amplifier. The light reception signal contains a pulsating component and a body movement component. The amplification circuit 162 or the control circuit 161 is capable of feeding a power supply voltage for operating the light-receiving part 16 at, e.g., reverse bias to the light-receiving part 16. In an instance in which the light-emitting part 14 is intermittently driven, the power supply to the light-receiving part 16 is intermittently fed, and the AC component is intermittently amplified. The amplification circuit 162 is formed on, e.g., the motherboard, and wiring between the amplification circuit 162 and the light-receiving part 16 is formed on, e.g., the substrate 11 shown in FIGS. 5A and 5B. The amplification circuit 162 may also have an amplifier for amplifying the light reception signal at a stage prior to the high-pass filter. In an instance in which the amplification circuit 162 has an amplifier, the amplifier is formed, e.g., on the substrate 11.

The A/D conversion circuit 163 shown in FIG. 13 converts an AC signal generated in the amplification circuit 162 into a digital signal (i.e., a first digital signal). The acceleration detecting part 166 shown in FIG. 13 detects, e.g., acceleration in three axes (i.e., an x-axis, a y-axis, and a z-axis) and generates an acceleration signal. Movement of the body (i.e., the arm), and therefore movement of the biological information measuring device, are reflected in the acceleration signal. The A/D conversion circuit 167 shown in FIG. 13 converts the acceleration signal generated in the acceleration detecting part 166 into a digital signal (i.e., a second digital signal).

The digital signal processing circuit 168 shown in FIG. 13 uses the second digital signal to remove or reduce the body movement component in the first digital signal. The digital signal processing circuit 168 may be formed with, e.g., an FIR filter or another adaptive filter. The digital signal processing circuit 168 inputs the first digital signal and the second digital signal into the adaptive filter and generates a filter output signal from which noise has been removed or which has reduced noise.

The pulse rate computation circuit 164 shown in FIG. 13 uses e.g., fast Fourier transform (or in a broader sense, scrambling Fourier transform) to perform a frequency analysis on the filter output signal. The pulse rate computation circuit 164 identifies a frequency that represents a pulsating component based on a result of the frequency analysis, and calculates a pulse rate.

Although a detailed description was made concerning the present embodiment as stated above, persons skilled in the art should be able to easily understand that various modifications are possible without substantially departing from the scope and effects as defined by the claims. Accordingly, all of such examples of modifications are to be included in the scope of the invention. For example, terms stated at least once together with different terms having broader sense or identical sense in the specification or drawings may be replaced with those different terms in any and all locations of the specification or drawings.

## Claims

1. A biological information detector, comprising:
a light-emitting part (14) for emitting light directed at a detection site (O) of a test subject;
a light-receiving part (16) for receiving light having biological information, the light being light emitted by the light-emitting part and reflected at the detection site; and
a contact part (19) formed from a material that is transparent with respect to a wavelength of the light emitted by the light-emitting part, the contact part having a surface (19A) that makes contact with the test subject and an opposing surface (19B) disposed opposite the contact surface (19A); wherein
the light-emitting part (14) is installed on the opposing surface (19B).

2. The biological information detector according to Claim 1, wherein
a first wiring for the light-emitting part (14) is arranged on the opposing surface, and
the light-emitting part (14) is installed on a surface of the first wiring.

3. The biological information detector according to Claim 1 or 2, wherein light emitted by the light-emitting part (14) includes a first light (R1) directed at the detection site (O) and a second light (R2) directed in a direction other than that of the detection site; and
the biological information detector further includes a first reflecting part (92) for reflecting the second light towards the detection site.

4. The biological information detector according to Claim 3, wherein
the first reflecting part (92) is secured to the opposing surface (19B).

5. The biological information detector according to Claim 4, wherein
the first reflecting part (92) is secured to the opposing surface (19B) with an insulating member (63-3, 64-3) interposed therebetween.

6. The biological information detector according to any of Claims 2 to 5 if dependent on Claim 2, further comprising:
a substrate (11) formed from a material that is transparent with respect to the wavelength of the light emitted by the light-emitting part, the substrate having a first surface (11A) and a second surface (11B) disposed opposite the first surface; wherein
a second wiring for the light-emitting part is arranged on the second surface; and
the first wiring is electrically connected to the second wiring with an electroconductive member (63-4, 64-4) interposed therebetween.

7. The biological information detector according to Claim 6, further comprising
a second reflecting part (18) for causing light having biological information from the detection site (O) to be reflected and guided towards the light-receiving part (16); wherein
the light-receiving part (16) is arranged on the first surface (11A); and
the substrate (11) is arranged between the second reflecting part (18) and the contact part (19).

8. A biological information measuring device, comprising:
the biological information detector according to Claim 1, and
a biological information measuring part for measuring the biological information from a light reception signal generated in the light-receiving part (16), wherein
the biological information is a pulse rate.
